# EUROPEAN PATENT SPECIFICATION

(11) **EP 3 807 241 B1**
(45) Date of publication and mention of the grant of the patent: **13.04.2022**
(21) Application number: 19729745.0
(22) Date of filing: 07.06.2019
(51) Int. Cl.: C07C 47/225

(54) **IMPROVEMENTS IN OR RELATING TO ORGANIC COMPOUNDS**
VERBESSERUNGEN AN ODER IM ZUSAMMENHANG MIT ORGANISCHEN VERBINDUNGEN
PERFECTIONNEMENTS APPORTÉS OU SE RAPPORTANT À DES COMPOSÉS ORGANIQUES

(30) Priority: 14.06.2018 GB 201809732
(43) Date of publication of application: 21.04.2021
(73) Proprietor: Givaudan SA, 1214 Vernier (CH)
(72) Inventor: COMPTON, Jeremy, 8302 Kloten (CH); LAUE, Heike, 8600 Duebendorf (CH); VOIROL, Francis, 8500 Frauenfeld (CH)
(74) Representative: Global Patents
(86) International application number: PCT/EP2019/064982
(87) International publication number: WO 2019/238568

(56) References cited:
- EP-A1- 2 578 671
- WO-A1-2014/180952
- WO-A1-2017/046071
- WO-A1-2018/167200

## Description

The present invention relates to a perfume composition comprising a first compound represented by formula (I), as specified in the independent claim.

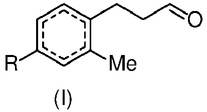

Compounds having muguet odor characteristics are very sought after as perfume ingredients. They are important constituents of floral bases and can act as harmonizers across many types of fragrance creations. Ingredients of this type are used widely in personal care and consumer care products as well as in fine perfumery to generate pleasant odors or to mask unpleasant odors.

An excellent perfume ingredient widely valued for its muguet odor note is Lilial^{™} (3-(4-tert-butylphenyl)-2-methylpropanal). Various analogues of this compound are disclosed in WO2014/180952. This compound has found wide use in various applications. However, its use is controversial in view of recent findings that it exhibits toxic effects on the reproductive organs of male rats and dogs. No effects were found in studies with mice, guinea-pigs and primates. Nevertheless, under the Global Harmonized System (GHS) Classification this compound was classified as a CMR2 material. For CMR category 2 materials, it is necessary to establish that quantities proposed for use are harmless to consumers. In view of the regulatory status of Lilial^{™}, it is being replaced with other perfume ingredients.

In vitro data using primary rat hepatocytes indicate that Lilial^{™} metabolites inhibit hepatic lipogenesis and gluconeogenesis (McCune et al., Arch. Biochem. Biophys. 1982, 214, 124-133). In particular 4-(tert-butyl)benzoic acid (TBBA) has been shown to disturb fatty acid synthesis and glucose synthesis. TBBA treatment also decreased CoA, acetyl-CoA and citrate levels. It was found that Lilial^{™} and TBBA rapidly and dose dependently transform to TBBA-CoA conjugates, which undergo accumulation to stable levels. The toxicity observed for Lilial^{™} is thus likely to be caused by disruption of CoA-dependent metabolic processes triggered by the metabolite TBBA.

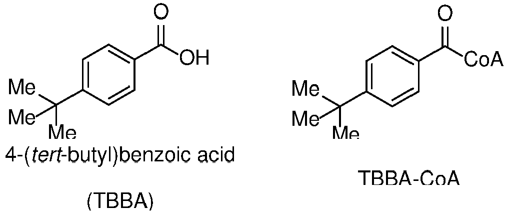

EP 2 578 671 A1 proposes 3-(4-isopropylcyclohex-1-en-1-yl)propanal as a replacement for Lilial^{™}. Taking into consideration that its structure is devoid of an aromatic ring, one would expect that the toxicity issues encountered with Lilial^{™}, and associated with TBBA-CoA conjugates, are no problem with this compound.

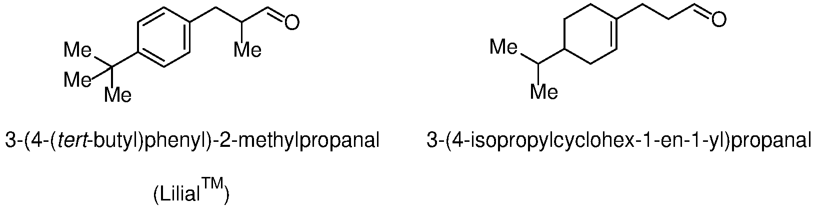

However, in vitro studies have now revealed that substantial accumulation of CoA-conjugates occurs with 3-(4-isopropylcyclohex-1-en-1-yl)propanal in plated rat hepatocytes. It can therefore not be excluded that some of the toxicity profile of Lilial^{™} might also be encountered with 3-(4-isopropylcyclohex-1-en-1-yl)propanal.

It is therefore a problem underlying the present invention to overcome the above-mentioned shortcomings in the prior art. In particular, it is a problem underlying the present invention to provide a novel compound that can be employed as a perfume ingredient with a muguet (lily of the valley) character. This compound is supposed to have reduced toxicity compared to 3-(4-isopropylcyclohex-1-en-1-yl)propanal and, in particular, should not attract CMR2 regulatory concerns.

These problems are solved by perfume compositions comprising a first compound according to claim 1. The compounds are represented by formula (I).

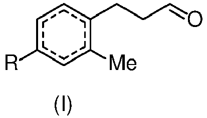

The half-doted double lines each represent a carbon-carbon single bond or a carbon-carbon double bond. The six-membered ring comprises exactly one endocyclic carbon-carbon double bond or exactly two endocyclic carbon-carbon double bonds. When the six-membered ring comprises exactly two endocyclic carbon-carbon double bonds, the endocyclic carbon-carbon double bonds are either in 1 ,3-relationship or in 1,4-relationship. R is selected from the group consisting of iso-propyl, iso-butyl, sec-butyl and tert-butyl.

In context of the present invention "carbon-carbon double bonds that are in 1,3-relationship" means that a first carbon atom that takes part in the first carbon-carbon double bond is connected over a carbon-carbon single bond to a second carbon atom that takes part in the second carbon-carbon double bond. "Carbon-carbon double bonds that are in 1 ,4-relationship", on the other hand, means that a first carbon atom that takes part in the first carbon-carbon double bond is connected over a carbon-carbon single bond to a second carbon atom, which is connected over a further carbon-carbon single bond to a third carbon atom that takes part in the second carbon-carbon double bond.

Compounds represented by formula (I) are employed as perfume ingredients in perfume compositions according to the present invention and in consumer products. They possess desirable muguet (lily of the valley) odor characteristics and are perceived and recognized by perfumers as being very reminiscent to Lilial^{™}. In particular, compounds represented by formula (I) have been shown to exhibit significantly decreased accumulation of CoA-conjugates in plated rat hepatocytes. They thus can serve as a replacement for Lilial^{™} with reduced toxicity. Most notably, such compounds do not attract regulatory concerns.

The improved toxicological profile of these compounds can be rationalized with the methyl group in α-position to the propanal substituent on the six-membered ring. It is surmised that this methyl substituent inhibits formation and accumulation of the corresponding CoA-conjugates.

The respective compounds can be represented by formula (II) or formula (III).

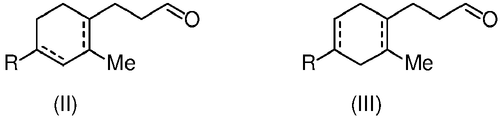

Compounds with such a structure have been found to have a particularly beneficial olfactory profile. In particular, these compounds show soft natural floralcy and have an aspect of benchmark dihydrocinnamic aldehydes (such as Lillial^{™} or cyclamenaldehyde), but enhance diffusivity and trail.

In preferred embodiments of the present invention, the compounds are represented by formula (IV) or formula (V).

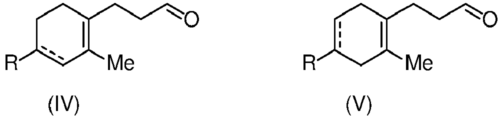

The compounds according to the present invention can be selected from the group consisting of 3-(4-(tert-butyl)-2-methylcyclohex-1-en-1-yl)propanal, 3-(4-isopropyl-2-methylcyclohex-1-en-1-yl)propanal,3-(4-(sec-butyl)-2-methylcyclohex-1-en-1-yl)propanal, 3-(4-isobutyl-2-methylcyclohex-1-en-1-yl)propanal, 3-(4-(tert-butyl)-6-methylcyclohex-3-en-1-yl)propanal, 3-(4-isopropyl-6-methylcyclohex-3-en-1-yl)propanal, 3-(4-(sec-butyl)-6-methylcyclohex-3-en-1-yl)propanal, 3-(4-isobutyl-6-methylcyclohex-3-en-1-yl)propanal, 3-(4-(tert-butyl)-2-methylcyclohex-3-en-1-yl)propanal, 3-(4-isopropyl-2-methylcyclohex-3-en-1-yl)propanal, 3-(4-(sec-butyl)-2-methylcyclohex-3-en-1-yl)propanal and 3-(4-isobutyl-2-methylcyclohex-3-en-1-yl)propanal.

In a particularly preferred embodiment of the present invention, the compound is 3-(4-isopropyl-2-methylcyclohex-1-en-1-yl)propanal or 3-(4-isobutyl-6-methylcyclohex-3-en-1-yl)propanal.

The present invention refers to a perfume composition comprising a compound as specified herein above. Such a perfume composition can be free of aryl-substituted alkanals, in particular aryl-substituted propanals, that are unsubstituted on the aryl ring at a position ortho to the substituent bearing the aldehyde functionality, in particular free of 3-(4-tert-butylphenyl)-2-methylpropanal.

The above-mentioned compounds may be present in the perfume composition in any amount depending on the particular olfactive effect that a perfumer wishes to achieve. In particular, the perfume composition according to the present invention may contain a compound as specified herein above in an amount of 0.1 to 100 % by weight of the composition.

A perfume composition according to the present invention comprises one or more additional fragrance ingredients.

The one or more additional fragrance ingredients are selected from the group consisting of 6-methoxy-2,6-dimethylheptan-1-al (methoxymelonal), 5,9-dimethyl-4,8-decadienal (geraldehyde), beta-methyl-3-(1-methylethyl)benzenepropanal (florhydral), octahydro-8,8-dimethylnaphthalene-2-carbaldehyde (cyclomyral), alphamethyl-1,3-benzodioxole-5-propionaldehyde (helional), 5-methyl-2-(1-methylbutyl)-5-propyl-1,3-dioxan (troenan), 3-(o-ethylphenyl)-2,2-dimethylpropionaldehyde (floralozone), farnesol, 3,7,11-trimethyldodeca-1,6,10-trien-3-ol, optionally as an isomeric mixture (nerolidol), 2-methyl-4-phenylbutan-2-ol (dimethylphenylethylcarbinol), 1-(1-hydroxyethyl)-4-(1-methylethyl)cyclohexane (optionally as a mixture of the diastereoisomers) (mugetanol), (4-methyl-3-pentenyl)cyclohexenecarbaldehyde (citrusal), cyclohexyl salicylate, 3-(p-(2-methylpropyl)phenyl)-2-methylpropionaldehyde (silvial) and 3-p-cumenyl-2-methylpropionaldehyde (cyclamenaldehyde).

A perfume composition according to the present invention need not be limited to the perfume ingredients listed above. Other perfume ingredients commonly used in perfumery may be employed, for example any of those ingredients described in "Perfume and Flavour Chemicals", S. Arctander, Allured Publishing Corporation, 1994, IL, USA.

Perfume ingredients that can be contained in perfume compositions according to the present invention are described above, but the mixture may not be limited to the stated ingredients. In particular, the perfume compositions may comprise adjuvants that are commonly employed in perfume compositions. The term "adjuvants" refers to ingredients that might be employed in a perfume composition for reasons not specifically related to the olfactive performance of said composition. For example, an adjuvant may be an ingredient that acts as an aid to processing a perfume ingredient or ingredients, or a composition containing said ingredient(s), or it may improve handling or storage of a perfume ingredient or composition containing same. It might also be an ingredient that provides additional benefits such as imparting color or texture. Furthermore, it might be an ingredient that imparts light resistance or chemical stability to one or more ingredients contained in a perfume ingredient or composition containing same. Examples of adjuvants include solvents and co-solvents, surfactants and emulsifiers, viscosity and rheology modifiers, thickening and gelling agents, preservative materials, pigments, dyestuffs and coloring matters, extenders, fillers and reinforcing agents, stabilizers against the detrimental effects of heat and light, bulking agents, acidulants, buffering agents and antioxidants.

The present invention also relates to a consumer product, in particular a personal care product or a household care product, comprising a perfume composition as specified herein above. As used herein, a "consumer product" means an article intended to be used or consumed in the form in which it is sold, and not intended for subsequent commercial manufacture or modification. In such a consumer product, the concentration of the compound according to the present invention can be 0.1 to 10 wt.-%, preferably 0.2 to 5 wt.-%, even more preferably 0.5 to 3 wt.-%.

The consumer product can be selected from the group consisting of a textile treatment product, an ironing aid, a laundry detergent, a cleaning product, in particular for hard and/or soft surfaces, a household cleaner, a personal care product, a laundry care product, a room fragrancer, an air freshener, a conditioner, a colorant, a fabric conditioner, a conditioning substrate, a pharmaceutical, a crop protection product, a polish, a food, a cosmetic product, a fertilizer, a building material, an adhesive, a bleach, a decalcifier, an autocare product, floorcare product, cookercare product, leathercare product or furniture care product, a scourer, a disinfectant, a fragrance and a mold remover.

Further advantages and particular features of the present invention become apparent form the following discussion of several examples.

### Example 1: Preparation of 3-(4-isopropyl-2-methylcyclo-hex-1-en-1-yl)propanal

### 1.1 General Remarks

"Usual workup" designates washing the organic layer containing the reaction product with water followed by aq. NaCl solution to adjust the pH to 6-7. The organic layer is then separated from the water phase, dried over MgSO₄, filtered, and the solvent evaporated in a rotary evaporator under reduced pressure.

### 1.2 Synthesis of 4-isopropyl-2-methylphenol

To a solution of 4-isopropylphenol (50.0 g, 0.37 mol) in acetonitrile (300 mL) were added successively under stirring at room temperature magnesium chloride (32.9 g, 0.55 mol), triethylamine (139 g, 1.38 mol) and para-formaldehyde (55.1 g, 1.84 mol). The temperature of the yellow mixture rose to 70°C and stirring was continued for 2 h. The mixture was then poured on 2M aq. HCI solution (500 mL) and the aqueous layer was extracted twice with MTBE (400 mL). After usual workup, 2-hydroxy-5-isopropylbenzaldehyde (57.4 g, 95%) was obtained as a yellow liquid.

A solution of this product (24.8 g, 151 mmol) in toluene (80 mL) was added rapidly at room temperature to Red-AI (60% wt/wt in toluene, 204 g, 605 mmol). The temperature rose to 65°C. The resulting mixture was heated to reflux for 12 h, then cooled to room temperature and poured on 2M aq. HCI solution (250 mL). After usual workup, a yellow liquid was obtained (21.3 g), which was distilled over a 7 cm Vigreux column at 0.05 mbar/65-71°C to yield 4-isopropyl-2-methylphenol (6.9 g, 30%) as a colorless liquid.

¹H-NMR (400 MHz, CDCl₃): 7.03 (d, *J*=2.2 Hz, 1 H), 6.98 (dd, *J*= 8.2, 2.3 Hz, 1 H), 6.74 (d, *J*= 8.1 Hz, 1 H), 4.76 (s, 1 H), 2.86 (dt, *J*= 13.8, 6.9 Hz, 1 H), 2.29 (s, 3 H), 1.26 (d, *J*= 6.8 Hz, 7 H). ¹³C-NMR (101 MHz, CDCl₃): 151.7 (s), 141.2 (s), 129.0 (d), 124.8 (d), 123.4 (s), 114.8 (d), 33.3 (d), 24.3 (2t), 15.9 (t).

### 1.3 Synthesis of 3-(4-isopropyl-2-methylcyclohex-1-en-1-yl)propan-1-ol

A solution of 4-isopropyl-2-methylphenol as prepared above (6.0 g, 39.9 mmol) in hexane (20 mL) was placed in a Premex autoclave and 10% Palladium on charcoal was added (400 mg). Hydrogenation was effected at 15 bar and 170°C. The mixture was filtered and the solvent evaporated to yield 4-isopropyl-2-methylcyclohexanol as a colorless oil (5.92 g, 95%). The product was dissolved in acetone (80 mL), the solution cooled to 5°C and Jones reagent (9.4 mL) was added dropwise, upon which the temperature rose to 20°C. Stirring was continued at room temperature for 30 min, then isopropanol (10 mL) was added and the mixture was poured on water (100 mL). After usual workup (extraction with hexane), 4-isopropyl-2-methylcyclohexanone was obtained as a colorless oil (5.4 g).

This product (5.0 g, 32.4 mmol) was dissolved in THF (80 mL) and the solution was cooled to 5°C. Then, a solution allyl magnesium chloride (2 M in THF, 24.3 mL, 48.6 mmol) was added dropwise over 4 min. After finished addition, the cooling bath was removed and stirring was continued at room temperature for 30 min, then the mixture was poured on water (100 mL). After usual workup (extraction with hexane), 1-allyl-4-isopropyl-2-methylcyclohexanol was obtained as a colorless oil (6.6 g).

Without purification, this product (5.6 g, 28.6 mmol) was suspended in water (40 mL) and sulfuric acid (97%, 14.4 mL) was added, upon which the temperature rose to 50°C. The mixture was heated to reflux for 8 h, then cooled and poured on water (100 mL). After usual workup (extraction with pentane, 1 initial washing with sat. aq. NaHCO₃ solution), 1-allyl-4-isopropyl-2-methylcyclohex-1-ene was obtained as a clear, colorless oil (3.79 g, 75%).

The crude product was dissolved in THF (30 mL) and the solution cooled to 5°C. A solution of 9-borabicyclo[3.3.1]nonane in THF (0.5 M, 51 mL, 51 mmol) was added dropwise. After completed addition, stirring was continued at room temperature for 1 h. The solution was cooled to 0°C and 2 M aq. NaOH solution (10.6 mL) was added, followed by the dropwise addition of aq. H₂O₂ solution (30%, 7.2 g), upon which the temperature rose to 30°C. After completed addition, the cooling bath was removed and the mixture was stirred for 30 min. Then it was poured on 2 M aq. HCI solution (40 mL). After usual workup, a clear yellow liquid was obtained (4.1 g) which was purified by flash chromatography over silica gel (eluent hexane/MTBE 4:1) to yield 3-(4-isopropyl-2-methylcyclohex-1-en-1-yl)propan-1-ol as a colorless oil (1.40 g, 34%). The product was 86% pure according to GC-MS analysis, the remainder being 3 not further elucidated isomeric compounds.

¹H-NMR (400 MHz, CDCl₃): 3.656 (t, *J*=6.48, 2H), 2.10 (t, *J*= 7. 7 Hz, 2H), 2.02-1.39 (m, 11H), 1.65 (s, 3H), 0.92 (d, *J*=6.72 Hz, 3H), 0.90 (d, *J*=6.72 Hz, 3H) ppm.

¹³C-NMR (101 MHz, CDCl₃): 129.3 (s), 126.5 (s), 63.2 (t), 40.9 (d), 35.7 (t), 32.4 (d), 31.2 (t), 30.3 (t), 29.4 (t), 26.8 (t), 19.8 (q), 19.7 (q), 19.1 (q) ppm.

GC/MS(EI): m/z (%): 196 (29) [M+], 153 (16), 135 (100), 109 (56), 93 (59), 79 (35), 67 (24), 55 (19), 41 (39).

### 1.4 Synthesis of 3-(4-isopropyl-2-methylcyclohex-1-en-1-yl)propanal

A solution of 3-(4-isopropyl-2-methylcyclohex-1-en-1-yl)propan-1-ol (1.3 g, 6.62 mmol) in dichloromethane (40 mL) was treated with pyridinium chlorochromate (2.0 g, 9.27 mmol). The mixture was stirred for 2 h, then filtered through a plug of silica. The clear yellow liquid obtained after removal of the solvent was purified by chromatography over silica gel (eluent hexane/MTBE 50:1) to yield 3-(4-isopropyl-2-methylcyclohex-1-en-1-yl)propanal as a colorless oil (0.75 g, 58%). The product was 87% pure according to GC-MS analysis, the remainder being 3 not further elucidated isomeric compounds.

¹H-NMR (400 MHz, CDCl₃): 9.77 (s, 1H), 2.51-2.46 (m, 2H), 2.39-2.30 (m, 2H), 20.2-1.02 (m, 8H), 1.64 (s, 3H), 0.91 (d, *J*=6.72 Hz, 3H), 0.89 (d, *J*=7.2 Hz, 3H) ppm.

¹³C-NMR (101 MHz, CDCl₃): 202.9 (s), 127.5 (2s), 42.6 (t), 40.7 (d), 35.7 (t), 32.3 (d), 30.1 (t), 26.7 (t), 25.7 (t), 19.9 (q), 19.7 (q), 19.1 (q) ppm.

GC/MS(EI): m/z (%): 194 (24) [M+], 176 (8), 151 (28), 133 (62), 107 (100), 91 (51), 79 (38), 67 (26), 55 (23), 41 (40), 31 (28).

### Example 2: Preparation of 3-(4-isobutyl-6-methylcyclohex-3-en-1-yl)propanal

### 2.1 Synthesis of ethyl-3-(4-isobutyl-6-methylcyclohex-3-en-1-yl)acrylate

Sodium hydride (7.1 g, 179 mmol) in THF (120 mL) was placed in a 350 mL sulfonation flask. A solution of triethylphosphonoacetate (40.1 g, 179 mmol) in THF (40 mL) was added dropwise over 10 minutes (the temperature was maintained with an ice bath between 22 and 35 °C). After the mixture was stirred for another 5 min, a solution of 4-isobutyl-6-methylcyclohex-3-ene-1-carbaldehyde (29.3 g, 163 mmol, prepared as described in WO 2012/020076 A1) in 40 mL of THF was added dropwise during 10 min, while the reaction temperature was maintained between 22 °C and 28 °C with an ice bath. After the addition was complete, the ice bath was removed and the mixture stirred for 1 h at room temperature. Then MTBE (150 mL) and cold diluted aq. HCI (200 mL) were added. The org. Phase was washed twice with water (100 mL) and once with satd. aq. NaCl (100 mL), dried over MgSO₄, filtered and concentrated in vacuo to afford 41.3g of a clear, pale orange liquid. The residue was distilled in vacuo (b.p. 110-118 °C, 0.2 mbar) to yield ethyl-3-(4-isobutyl-6-methylcyclohex-3-en-1-yl)acrylate (30.0g, 73%), which was directly used without further analysis in the next step.

### 2.2 Synthesis of 3-(4-Isobutyl-6-methylcyclohex-3-en-1-yl)propanal

A catalytic amount of Raney nickel was suspended into a solution of ethyl-3-(4-isobutyl-6-methylcyclohex-3-en-1-yl)acrylate (30.0g, 120 mmol) in ethanol (150 mL). The mixture was hydrogenated under slight pressure of 0.4 bar at room temperature. After 3069 mL of hydrogen were consumed, the mixture was filtered and concentrated in vacuo to dryness to afford 30.2 g of a colorless oil. A part of this residue (5.0 g, 19.81 mmol) was dissolved in toluene (50 mL) and cooled to -75 °C. Diisobutylaluminum hydride (39.6 ml 1M in toluene) was added dropwise over 15 minutes (the temperature rose to -68 °C). After completion of the addition, the cold bath was removed and the mixture stirred for 1 h, cooled to -75 ° C and another 19.8ml of diisobutylaluminum hydride (1M in toluene) was added dropwise. The cold bath was removed and the mixture stirred for 45min. The reaction mixture was then added to aq. HCI (2M, 150 mL), was extracted with MTBE (2 x 150mL) and the combined org. phases were washed with water (2 x 100 mL) and satd. aq. NaCl (100mL), dried (MgSO₄) and concentrated to yield a clear slightly yellowish oil of crude 3-(4-isobutyl-6-methylcyclohex-3-en-1-yl)propan-1-ol (3.04 g, 73 %). This material was diluted with CH₂Cl₂ (50 mL) and PCC (4.36g, 20.23 mmol) was added in one portion and the mixture was stirred for 1 h, filtered over Florisil (100-200 mesh) and evaporated in vacuo. The residue was purified by chromatography over silica gel (MTBE:hexane, 1:50) to yield 3-(4-isobutyl-6-methylcyclohex-3-en-1-yl)propanal (1.81 g, 60%) as a clear colorless oil.

¹H NMR (400 MHz, CDCl₃): 9.80 (s, 1H), 5.31 (bs, 1H), 2.53-2.40 (m, 2H), 2.12-1.25 (m, 11H), 0.98 (d, *J*=6.4 Hz, 3H), 0.86 (d, *J*= 6.5 Hz, 3H), 0.84 (d, *J*= 6.5 Hz, 3H) ppm.

¹³C NMR (101 MHz, CDCl₃): 203.3 (d), 136.5 (s), 120.8 (d), 47.8 (t), 41.8 (t), 38.9 (d), 36.7 (t), 33.1 (d), 30.4 (t), 36.3 (d), 25.8 (t), 23.0 (q), 22.7 (q), 19.9 (q) ppm.

GC/MS(EI): m/z (%): 208 (13) [M+], 190 (61), 175 (29), 164 (14), 147 (81), 133 (100), 119 (49), 107 (83), 93 (92), 79 (63), 67 (43), 57 (61), 41 (58), 29 (15).

### Example 3: Preparation of 3-(4-isobutylcyclohex-3-en-1-yl)propanal (not according to the present invention)

This compound was produced from 4-isobutylcyclohex-3-ene-1-carbaldehyde (WO 2012/020076 A1) according the procedure of Example 2.

¹H NMR (400 MHz, CDCl₃): 9.78 (s, 1H), 5.32 (bs, 1H), 2.49-2.44 (m, 2H), 2.12-1.44 (m, 11H), 1.28-1.18 (m, 1H), 0.84 (d, *J*= 6.5 Hz, 3H), 0.82 (d, *J*= 6.5 Hz, 3H) ppm.

¹³C NMR (101 MHz, CDCl₃): 203.1 (d), 137.3 (s), 121.3 (d), 47.9 (t), 42.0 (t), 33.6 (d), 32.1 (t), 29.5 (t), 28.4 (t), 28.7 (t), 26.4 (d), 23.0 (q), 22.7 (q) ppm.

### Example 4: Odor descriptions and GC odor thresholds

**Table 1**

| **Compound Name** | **Odor Description** | **GC Odor Threshold** |
|---|---|---|
| 3-(4-isopropyl-2-methylcyclo-hex-1-en-1-yl)propanal | aldehydic, green, fatty, muguet, bourgeonal | 0.022 ng/L |
| 3-(4-isobutyl-6-methylcyclo-hex-3-en-1-yl)propanal | aldehydic, green, floral, fatty, orris | n.a. |

### Example 5: A green floral muguet accord for use at 3% concentration in all-purpose cleaner applications containing 3-(4-isopropyl-2-methylcyclo-hex-1-en-1-yl)propanal

**Table 2**

| **CAS Number** | **Ingredient Name** | **Amount** | **Amount** |
|---|---|---|---|
| | | | |
| 140-11-4 | ACET BENZYLE | 180 | |
| 98-86-2 | ACETOPHENONE EXTRA @ 10% DEP | 1 | |
| 60-12-8 | ALC PHENYL ETHYLIQUE | 100 | |
| 101-86-0 | ALD A HEXYL CINNAMIQUE | 60 | |
| 100-52-7 | BENZALDEHYDE | 1 | |
| 1655500-83-6 | ROSYFOLIA | 4 | |
| 926-50-1 | MAHONIAL | 30 | |
| 1637294-12-2 | NYMPHEAL | 10 | |
| 93-58-3 | BENZOATE METHYLE | 1 | |
| 106-22-9 | CITRONELLOL EXTRA | 80 | |
| 68039-49-6 | CYCLAL C | 1 | |
| 8000-48-4 | EUCALYPTUS CITRIOD ESS BRES | 1 | |
| 107-75-5 | HYDROXYCITRONELLAL SYNT | 60 | |
| 120-72-9 | INDOLE PUR | 5 | |
| 16409-43-1 | ROSE OXI DE CO @10% DPG | 6 | |
| 84-66-2 | PHTALATE DIETHYLE | | |
| 106-24-1 | GERANIOL 980 | 40 | |
| 8000-41-7 | TERPI NEOL PUR | 40 | |
| | | | |
| | 3-(4-isopropyl-2-methylcyclo-hex-1-en-1-yl)propanal | | 40 |
| | | | |
| 25265-71-8 | DI PROPYLENE GLYCOL | 380 | 340 |
| | | | |
| | Total | 1000 | 100 |

The addition of 3-(4-isopropyl-2-methylcyclo-hex-1-en-1-yl)propanal reinforces the natural green muguet character of the composition. It also boosts the lift of the perfume when assessed on neat finish product, and when the all-purpose cleaner is freshly diluted in water, also when mopping.

### Example 6: CoA conjugate formation using plated rat hepatocvtes

### 6.1 General

Short-term monolayer cultures of cryopreserved primary hepatocytes from rat were prepared in collagen coated 48-well plates to perform metabolic assays in a kinetic manner for up to 22 h.

### 6.2 Preparation of monolayer cultures of rat hepatocytes

Palatable primary male rat hepatocytes (Sprague-Dawley, Grade P pooled cryopreserved hepatocytes, for plated assays) and supplements were purchased from Biopredic International (Saint Grégoire, France). WEM (with Phenol Red, with GlutaMAX^{™}, without HEPES) was purchased from Gibco^{™} (ThermoFisherScientific).

Cells were thawed following the supplier's protocol and seeded at a density of 450,000 cells/mL in 0.25 mL seeding medium (WEM supplemented with fetal bovine serum, Dexamethasone, penicillin-streptomycin, insulin, GlutaMAX^{™} and HEPES) using 48-well plates which were coated with collagen (Collagen I Rat Protein, Tail; Gibco^{™}, ThermoFisherScientific) and incubated for 4-5 h to facilitate attachment. Then, the medium was changed to culturing medium (WEM supplemented with penicillin, streptomycin, insulin, hydrocortisone without serum) to prevent detachment of cells.

### 6.3 Formation of acyl-CoA conjugates in plated rat and human hepatocytes

Test chemicals were dissolved in methanol (80-90% methanol) and directly added with the culture medium (0.8-0.9% final methanol concentration). For structure-activity studies, final concentration of test chemicals was 50 µM or 5 µM. Cells were incubated for 0.5-22 h in the presence of test chemicals. To stop the reactions, the culture medium was removed and 0.5 M citric acid (pH 2.0; 60 µL) and cold acetonitrile (60 µL) containing 2 µM decanoyl CoA as internal standard were added to each well. The attached cells were removed and disrupted by scraping the well with a pipette tip and by pipetting up and down several times, transferred in 1.5 mL tubes and frozen at -80°C. The cell lysates were defrozen, centrifuged (21,000 x g, 5 min, RT), and the supernatant was diluted with 1.36 mL Tris-buffer (4 mM) containing KCI (6 mM), MgCl₂ (0.3 mM) and n-heptadecanoyl CoA as internal standard (0.2 µM). Samples were neutralized by addition of NaOH (42 µL of 1 M NaOH) and loaded onto solid phase extraction cartridges (OASIS HLB µEIution plate, Waters). Columns were washed with 200 µL of ammonium solution (1 M), and samples eluted sequentially with 50 µL acetonitrile and 50 µL water.

### 6.4 Detection of CoA conjugates by LC-HRMS

CoA conjugates were analyzed with high resolution LC-MS (LC-HRMS) on a Dionex UltiMate 3000 RS HPLC system coupled to a Q-Exactive orbitrap mass spectrometer (Thermo Scientific, Reinach, Switzerland) with electrospray ionization (ESI) in both positive and negative ionization mode. For liquid chromatography separation an Agilent Zorbax 300Extend-C18 column with dimensions 2.1 mm x 50 mm and particle size of 3.5 µm with a 2.1 mm x 10 mm pre-column of the same material was used. The flow rate was 0.4 mL/min. Eluent A consists of water containing 0.0025% ammonia (pH around 9.8) and eluent B consists of acetonitrile containing 0.0025% ammonia. A linear gradient was run from 95% eluent A (hold for 1 min) to 100% eluent B within 6 min (hold for 1 min), back to 95% eluent A within 0.5 min followed by 1.5 min equilibration time. The injection volume of the sample was 10 µl. The mass resolution of the HR-MS spectra was set to 70,000. The mass accuracy was <5ppm. Data-dependent high resolution product ion spectra (HR-MS/MS) were recorded at a resolution of 17,500. Ion source parameters adjusted were as follows: sheath gas flow (30 arbitrary unit), auxiliary gas flow (10 arbitrary unit), capillary temperature (270°C), and source voltage (4kV in positive mode, -3kV in negative mode). Fragmentation was obtained from dissociation in an octopole collision cell using higher energy collision dissociation settings at 35 and 45 (arbitrary unit). The mass scan range was set from 120 to 1800 m/z.

Calibration standards were prepared with CoA, acetyl-CoA, benzoyl-CoA, TBBA-CoA, and octanoyl-CoA to determine the concentrations of CoA conjugates. Decanoyl-CoA and heptadecanoyl-CoA were used as internal standards. The limit of detection with this method is 0.005 µM for CoA conjugates.

### 6.5. Results

### 6.5.1 p-alkyl benzoyl-Coenzyme A formation from TBBA and BMHCA in plated rat hepatocytes

In rat hepatocytes, TBBA is rapidly transformed to p-tert-butyl-benzoyl-CoA. This metabolite accumulates to stable levels within 0.5-4 h. Almost identical results were obtained with BMHCA as test chemical compared to TBBA. TBBA appears to be formed rapidly from BMHCA and is conjugated to CoA.

### 6.5.2 Formation of benzoyl-Coenzyme A conjugates for BMHCA-like materials

In addition to TBBA and BMHCA other aldehydes were tested in the plated rat hepatocytes assay. These experiments were performed at a test concentration of 50 µM. All data are expressed in % relative to TBBA-CoA-formation from BMHCA at the given time point and at the corresponding concentration of the test chemical. This reference was tested in each experiment to normalize for batch-to-batch and experimental variation. Table 3 summarizes the chemical structures and the outcome of this metabolism assay in plated rat hepatocytes.

**Table 3**

| **Test Chemical** | | **CoA Conjugates Detected in Plated Rat Hepatocytes (% of TBBA-CoA ex BMHCA; NF = not found)** | | |
|---|---|---|---|---|
| **Compound Name** | **Structure** | **0.5 h** | **4 h** | **22 h** |
| (3-(4-tert-butylphenyl)-2-methylpropanal; BMHCA) (Lilial^{™}) | 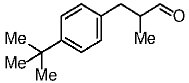 | 100 | 100 | 100 |
| (3-(4-isopropylcyclohex-1-en-1-yl)propanal) | 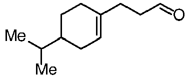 | 124 | 79 | 31 |
| 3-(4-isopropyl-2-methylcyclo-hex-1-en-1-yl)propanal | 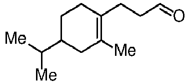 | 62 | 28 | 9 |
| 3-(4-isobutylcyclohex-3-en-1-yl)propanal | 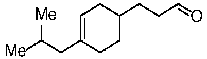 | 74 | 45 | 29 |
| 3-(4-isobutyl-6-methylcyclohex-3-en-1-yl)propanal | 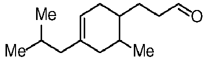 | 86 | 31 | NF |

For 3-(4-isopropylcyclohex-1-en-1-yl)propanal accumulation of CoA conjugates was observed, with a sustained accumulation (31% relative to BMHCA) after 22 h of incubation. The following Table 4 gives an overview of the CoA conjugates that were identified.

**Table 4**

| **Test Chemical: 3-(4-isopropylcyclo-hex-1-en-1-y))propana)** | | **CoA Conjugates Detected in Plated Rat Hepatocytes (% of TBBA-CoA ex BMHCA; NF = not found)** | | |
|---|---|---|---|---|
| **CoA Conjugate Name** | **Structure** | **0.5 h** | **4 h** | **22 h** |
| 3-(4-isopropylcyclohex-1-en-1-yl)propanoic acid-CoA | 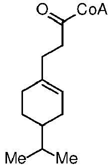 | 53 | 26 | NF |
| 3-(4-isopropylcyclohexa-1 ,5-dien-1-yl)propanoic acid-CoA | 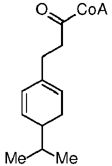 | 36 | 17 | NF |
| 4-isopropylcyclohex-1-ene-1-carboxylic acid-CoA | 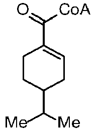 | 23 | 26 | 24 |
| 4-isopropylcyclohexane-1-carboxylic acid-CoA | 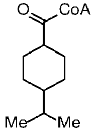 | 12 | 10 | 7 |
| Total Amount | | 124 | 79 | 31 |

On the other hand, for 3-(4-isopropyl-2-methylcyclohex-1-en-1-yl)propanal significantly reduced accumulation of CoA conjugates was found (only a total of 9% at the 22 h time point). The following Table 5 gives an overview of the CoA conjugates that were identified.

**Table 5**

| **Test Chemical: 3-(4-isopropyl-2-methylcyclohex-1-en-1-yl) propanal** | | **CoA Conjugates Detected in Plated Rat Hepatocytes (% of TBBA-CoA ex BMHCA; NF = not found)** | | |
|---|---|---|---|---|
| **CoA Conjugate Name** | **Structure** | **0.5 h** | **4 h** | **22 h** |
| 3-(4-isopropyl-2-methylcyclohex-1-en-1-yl)propanoic acid-CoA | 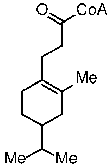 | 22 | 10 | 3 |
| 3-(4-isopropyl-2-methylcyclohexa-1,5-dien-1-yl)propanoic acid-CoA | 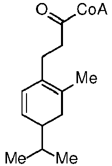 | 34 | 13 | 3 |
| 3-(4-isopropyl-2-methylphenyl)propanoic acid-CoA | 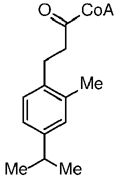 | 2 | 2 | NF |
| 4-isopropyl-2-methylcyclohex-1-ene-1-carboxylic acid-CoA | 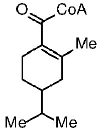 | 4 | 3 | 3 |
| Total Amount | | 62 | 28 | 9 |

Furthermore, 3-(4-isobutyl-cyclohex-3-en-1-yl)propanal was tested (Table 6). This compound shares some structural features with 3-(4-isopropylcyclohex-1-en-1-yl)propanal, as it is also a cyclohexenylpropanal, which is unsubstituted on the six-membered ring in α-position to the propanal substituent. In the experiment, similar amounts of CoA conjugates (29% at the 22 h time point) were observed as with 3-(4-isopropylcyclohex-1-en-1-yl)propanal. But the main conjugate detected was the aromatic 4-isobutylbenzoic acid-CoA.

**Table 6**

| **Test Chemical: 3-(4-isobutyl-cyclohex-3-en-1-yl) propanal** | | **CoA Conjugates Detected in Plated Rat Hepatocytes (% of TBBA-CoA ex BMHCA; NF = not found)** | | |
|---|---|---|---|---|
| **CoA Conjugate Name** | **Structure** | **0.5 h** | **4 h** | **22 h** |
| 3-(4-isobutylcyclohex-3-en-1-yl)propanoic acid-CoA | 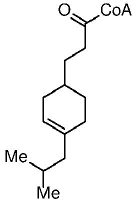 | 19 | 2 | 3 |
| 3-(4-isobutylcyclohexa-1,3-dien-1-yl)propanoic acid-CoA | 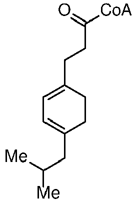 | 12 | 2 | NF |
| 4-isobutylcyclohex-3-ene-1-carboxylic acid-CoA | 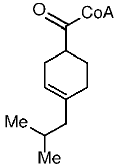 | 23 | 10 | 5 |
| 4-isobutylcyclohexa-1,3-diene-1-carboxylic acid-CoA | 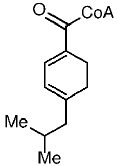 | 8 | 8 | NF |
| 4-isobutylbenzoic acid-CoA | 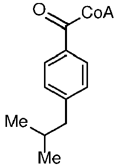 | 12 | 23 | 21 |
| Total Amount | | 74 | 45 | 29 |

On the other hand, for 3-(4-isobutyl-6-methylcyclohex-3-en-1-yl)propanal, which bears a methyl group on the six-membered ring in α-position to the propanal substituent, no CoA conjugates were detected after 22 h. The following Table 7 gives an overview of the CoA conjugates that were identified.

**Table 7**

| **Test Chemical: 3-(4-isobutyl-6-methyl-cyclohex-3-en-1-yl) propanal** | | **CoA Conjugates Detected in Plated Rat Hepatocytes (% of TBBA-CoA ex BMHCA; NF = not found)** | | |
|---|---|---|---|---|
| **CoA Conjugate Name** | **Structure** | **0.5 h** | **4 h** | **22 h** |
| 3-(4-isobutyl-6-methylcyclohex-3-en-1-yl)propanoic acid | 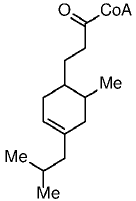 | 58 | 19 | NF |
| 3-(4-isobutyl-6-methylcyclohexa-1,3-dien-1-yl)propanoic acid-CoA | 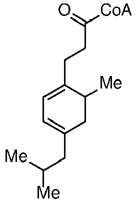 | 21 | 8 | NF |
| 3-(4-isobutyl-2-methylcyclohexyl)propanoic acid-CoA | 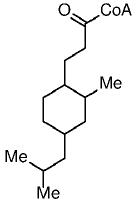 | 3 | NF | NF |
| 4-isobutyl-6-methylcyclohex-3-ene-1-carboxylic acid-CoA | 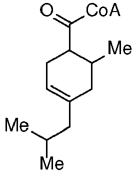 | 4 | 4 | NF |
| Total Amount | | 86 | 31 | NF |

**Table 6**

| **Test Chemical: 3-(4-isobutyl-cyclohex-3-en-1-yl)propanal** | | **CoA Conjugates Detected in Plated Rat Hepatocytes (% of TBBA-CoA ex BMHCA; NF = not found)** | | |
|---|---|---|---|---|
| **CoA Conjugate Name** | **Structure** | **0.5 h** | **4 h** | **22 h** |
| 3-(4-isobutylcyclohex-3-en-1-yl)propanoic acid-CoA | 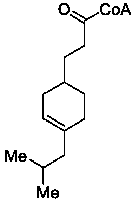 | 19 | 2 | 3 |
| 3-(4-isobutylcyclohexa-1,3-dien-1-yl)propanoic acid-CoA | 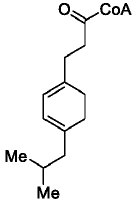 | 12 | 2 | NF |
| 4-isobutylcyclohex-3-ene-1-carboxylic acid-CoA | 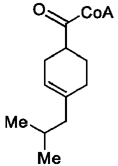 | 23 | 10 | 5 |
| 4-isobutylcyclohexa-1,3-diene-1-carboxylic acid-CoA | 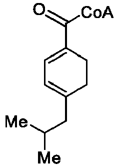 | 8 | 8 | NF |
| 4-isobutylbenzoic acid-CoA | 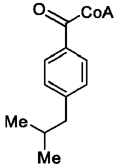 | 12 | 23 | 21 |
| Total Amount | | 74 | 45 | 29 |

On the other hand, for 3-(4-isobutyl-6-methylcyclohex-3-en-1-yl)propanal, which bears a methyl group on the six-membered ring in α-position to the propanal substituent, no CoA conjugates were detected after 22 h. The following Table 7 gives an overview of the CoA conjugates that were identified.

**Table 7**

| **Test Chemical: 3-(4-isobutyl-6-methyl-cyclohex-3-en-1-yl)propanal** | | **CoA Conjugates Detected in Plated Rat Hepatocytes (% of TBBA-CoA ex BMHCA; NF = not found)** | | |
|---|---|---|---|---|
| **CoA Conjugate Name** | **Structure** | **0.5 h** | **4 h** | **22 h** |
| 3-(4-isobutyl-6-methylcyclohex-3-en-1-yl)propanoic acid | 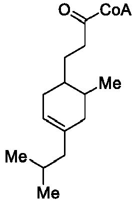 | 58 | 19 | NF |
| 3-(4-isobutyl-6-methylcyclohexa-1,3-dien-1-yl)propanoic acid-CoA | 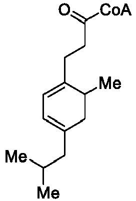 | 21 | 8 | NF |
| 3-(4-isobutyl-2-methylcyclohexyl)propanoic acid-CoA | 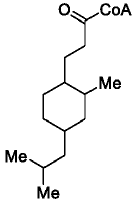 | 3 | NF | NF |
| 4-isobutyl-6-methylcyclohex-3-ene-1-carboxylic acid-CoA | 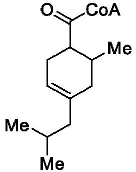 | 4 | 4 | NF |
| Total Amount | | 86 | 31 | NF |

## Claims

1. A perfume composition comprising a first compound represented by formula (I) 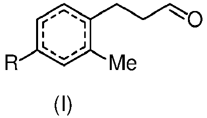
wherein the half-doted double lines each represent a carbon-carbon single bond or a carbon-carbon double bond, wherein the six-membered ring comprises exactly one endocyclic carbon-carbon double bond or exactly two endocyclic carbon-carbon double bonds, wherein, when the six-membered ring comprises exactly two endocyclic carbon-carbon double bonds, the endocyclic carbon-carbon double bonds are either in 1 ,3-relationship or in 1,4-relationship, wherein R is selected from the group consisting of iso-propyl, iso-butyl, sec-butyl and tert-butyl,
the perfume composition comprising one or more additional fragrance ingredients selected from the group consisting of 6-methoxy-2,6-dimethylheptan-1-al, 5,9-dimethyl-4,8-decadienal, beta-methyl-3-(1-methylethyl)benzenepropanal, octahydro-8,8-dimethylnaphthalene-2-carbaldehyde, alpha-methyl-1,3-benzodioxole-5-propionaldehyde, 5-methyl-2-(1-methylbutyl)-5-propyl-1,3-dioxan, 3-(o-ethylphenyl)-2,2-dimethylpropionaldehyde, farnesol, 3,7,11-trimethyldodeca-1, 6, 10-trien-3-ol, optionally as an isomeric mixture, 2-methyl-4-phenylbutan-2-ol, 1-(1-hydroxyethyl)-4-(1-methylethyl)cyclohexane (optionally as a mixture of the diastereoisomers), (4-methyl-3-pentenyl)cyclohexenecarbaldehyde, cyclohexyl salicylate, 3-(p-(2-methylpropyl)phenyl)-2-methylpropionaldehyde and 3-p-cumenyl-2-methylpropionaldehyde.

2. A perfume composition according to claim 1, wherein the first compound is represented by formula (II)

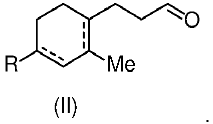

3. A perfume composition according to claim 1, wherein the first compound is represented by formula (III)

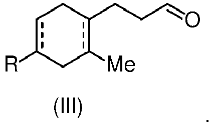

4. A perfume composition according to claim 2, wherein the first compound is represented by formula (IV)

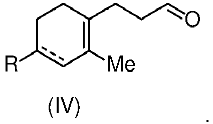

5. A perfume composition according to claim 3, wherein the first compound is represented by formula (V)

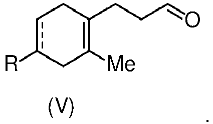

6. A perfume composition according to claim 1, wherein the first compound is selected from the group consisting of 3-(4-(tert-butyl)-2-methylcyclohex-1-en-1-yl)propanal, 3-(4-isopropyl-2-methylcyclohex-1-en-1-yl)propanal, 3-(4-(sec-butyl)-2-methylcyclohex-1-en-1-yl)propanal, 3-(4-isobutyl-2-methylcyclohex-1-en-1-yl)propanal, 3-(4-(tert-butyl)-6-methylcyclohex-3-en-1-yl)propanal, 3-(4-isopropyl-6-methylcyclohex-3-en-1-yl)propanal, 3-(4-(sec-butyl)-6-methylcyclohex-3-en-1-yl)propanal, 3-(4-isobutyl-6-methylcyclohex-3-en-1-yl)propanal, 3-(4-(tert-butyl)-2-methylcyclohex-3-en-1-yl)propanal, 3-(4-isopropyl-2-methylcyclohex-3-en-1-yl)propanal, 3-(4-(sec-butyl)-2-methylcyclohex-3-en-1-yl)propanal and 3-(4-isobutyl-2-methylcyclohex-3-en-1-yl)propanal.

7. A perfume composition according to one of claims 1 to 7 that is free of aryl-substituted alkanals, in particular aryl-substituted propanals, that are unsubstituted on the aryl ring at a position ortho to the substituent bearing the aldehyde functionality, in particular free of 3-(4-tert-butylphenyl)-2-methylpropanal.

8. A consumer product, in particular a personal care product or a household care product, comprising a perfume composition according to one of claims 1 to 7.

## Patentansprüche

1. Parfümzusammensetzung, umfassend eine erste Verbindung, die durch Formel (I) wiedergegeben wird: 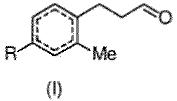
wobei die halb gestrichelten doppelten Linien jeweils für eine Kohlenstoff-Kohlenstoffbindung Einfachbindung oder eine Kohlenstoff-Kohlenstoff-Doppelbindung stehen, wobei der sechsgliedrige Ring genau eine endocyclische Kohlenstoff-Kohlenstoff-Doppelbindung oder genau zwei endocyclische Kohlenstoff-Kohlenstoff-Doppelbindungen umfasst, wobei dann, wenn der sechsgliedrige Ring genau zwei endocyclische Kohlenstoff-Kohlenstoff-Doppelbindungen umfasst, die endocyclischen Kohlenstoff-Kohlenstoff-Doppelbindungen entweder in 1,3-Beziehung oder in 1,4-Beziehung stehen, wobei R aus der Gruppe bestehend aus Isopropyl, Isobutyl, sec-Butyl und tert-Butyl ausgewählt ist,
wobei die Parfümzusammensetzung einen oder mehrere zusätzliche Duftstoffbestandteile aus der Gruppe bestehend aus 6-Methoxy-2,6-dimethylheptan-1-al, 5,9-Dimethyl-4,8-decadienal, beta-Methyl-3-(1-methylethyl)benzolpropanal, Octahydro-8,8-dimethylnaphthalin-2-carbaldehyd, alpha-Methyl-1,3-benzodioxol-5-propionaldehyd, 5-Methyl-2-(1-methylbutyl)-5-propyl-1,3-dioxan, 3-(o-Ethylphenyl)-2,2-dimethylpropionaldehyd, Farnesol, 3, 7, 11-Trimethyldodeca-1, 6, 10-trien-3-ol, gegebenenfalls als Isomerengemisch, 2-Methyl-4-phenyl-butan-2-ol, 1-(1-Hydroxyethyl)-4-(1-methylethyl)-cyclohexan (gegebenenfalls als Gemisch von Diastereoisomeren), (4-Methyl-3-pentenyl)cyclo-hexencarbaldehyd, Cyclohexylsalicylat, 3-(p-(2-Methylpropyl)phenyl)-2-methylpropionaldehyd und 3-p-Cumenyl-2-methylpropionaldehyd umfasst.

2. Parfümzusammensetzung nach Anspruch 1, wobei die erste Verbindung durch Formel (II) wiedergegeben wird:

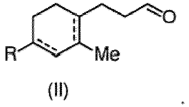

3. Parfümzusammensetzung nach Anspruch 1, wobei die erste Verbindung durch Formel (III) wiedergegeben wird:

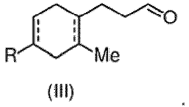

4. Parfümzusammensetzung nach Anspruch 2, wobei die erste Verbindung durch Formel (IV) wiedergegeben wird:

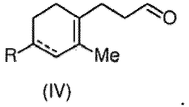

5. Parfümzusammensetzung nach Anspruch 3, wobei die erste Verbindung durch Formel (V) wiedergegeben wird:

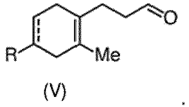

6. Parfümzusammensetzung nach Anspruch 1, wobei die erste Verbindung aus der Gruppe bestehend aus 3-(4-(tert-Butyl)-2-methylcyclohex-1-en-1-yl)propanal, 3-(4-Isopropyl-2-methylcyclohex-1-en-1-yl)-propanal, 3-(4-(sec-Butyl)-2-methylcyclohex-1-en-1-yl)propanal, 3-(4-Isobutyl-2-methylcyclohex-1-en-1-yl)propanal, 3-(4-(tert-Butyl)-6-methylcyclohex-3-en-1-yl)propanal, 3-(4-Isopropyl-6-methylcyclohex-3-en-1-yl)propanal, 3-(4-(sec-Butyl)-6-methylcyclohex-3-en-1-yl)propanal, 3-(4-Isobutyl-6-methylcyclohex-3-en-1-yl)propanal, 3-(4-(tert-Butyl)-2-methylcyclohex-3-en-1-yl)-propanal, 3-(4-Isopropyl-2-methylcyclohex-3-en-1-yl)propanal, 3-(4-(sec-Butyl)-2-methylcyclohex-3-en-1-yl)propanal und 3-(4-Isobutyl-2-methylcyclo-hex-3-en-1-yl)propanal ausgewählt ist.

7. Parfümzusammensetzung nach einem der Ansprüche 1 bis 7, die frei von arylsubstituierten Alkanalen, insbesondere arylsubstituierten Propanalen, die am Arylring an einer zu dem die Aldehydfunktionalität tragenden Substituenten ortho-ständigen Position unsubstituiert sind, insbesondere frei von 3-(4-tert-Butylphenyl)-2-methylpropanal, sind.

8. Konsumprodukt, insbesondere Körperpflegeprodukt oder Haushaltspflegeprodukt, umfassend eine Parfümzusammensetzung nach einem der Ansprüche 1 bis 7 .

## Revendications

1. Composition de parfum comprenant un premier composé représenté par la formule (I) 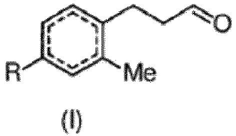
les lignes doubles en semi-pointillés représentant chacune une simple liaison carbone-carbone ou une double liaison carbone-carbone, le cycle à 6 chaînons comprenant exactement une double liaison carbone-carbone endocyclique ou exactement deux doubles liaisons carbone-carbone endocycliques, dans laquelle, lorsque le cycle à 6 chaînons comprend exactement deux doubles liaisons carbone-carbone endocycliques, les doubles liaisons carbone-carbone endocycliques sont soit dans une relation 1,3, soit dans une relation 1, 4, R étant choisi dans le groupe constitué par iso-propyle, iso-butyle, sec-butyle et tert-butyle,
la composition de parfum comprenant un ou plusieurs ingrédients de fragrance supplémentaires choisis dans le groupe constitué par le 6-méthoxy-2, 6-diméthylheptan-1-al, le 5, 9-diméthyl-4, 8-décadiénal, le bêta-méthyl-3-(1-méthyléthyl)benzènepropanal, l'octahydro-8, 8-diméthylnaphtalène-2-carbaldéhyde, l'alpha-méthyl-1,3-benzodioxole-5-propionaldéhyde, le 5-méthyl-2-(1-méthylbutyl)-5-propyl-1,3-dioxanne, le 3-(o-éthylphényl)-2,2-diméthylpropionaldéhyde, le farnésol, le 3, 7, 11-triméthyldodéca-1, 6, 10-trién-3-ol, éventuellement en tant que mélange isomérique, le 2-méthyl-4-phénylbutan-2-ol, le 1-(1-hydroxyéthyl)-4-(1-méthyléthyl)cyclohexane (éventuellement en tant que mélange des diastéréoisomères), le (4-méthyl-3-pentényl)cyclohexènecarbaldéhyde, le salicylate de cyclohexyle, le 3-(p-(2-méthylpropyl)phényl)-2-méthylpropionaldéhyde et le 3-p-cuményl-2-méthylpropionaldéhyde.

2. Composition de parfum selon la revendication 1, le premier composé étant représenté par la formule (II)

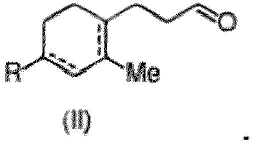

3. Composition de parfum selon la revendication 1, le premier composé étant représenté par la formule (III)

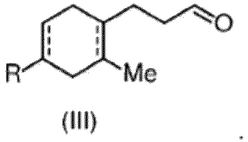

4. Composition de parfum selon la revendication 2, le premier composé étant représenté par la formule (IV)

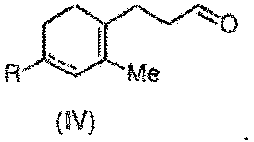

5. Composition de parfum selon la revendication 3, le premier composé étant représenté par la formule (V)

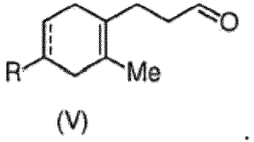

6. Composition de parfum selon la revendication 1, le premier composé étant choisi dans le groupe constitué par le 3-(4-(tert-butyl)-2-méthylcyclohex-1-én-1-yl)propanal, le 3-(4-isopropyl-2-méthylcyclohex-1-én-1-yl)propanai, le 3-(4-(sec-butyl)-2-méthylcyclohex-1-én-1-yl)propanal, le 3-(4-isobutyl-2-méthylcyclohex-1-én-1-yl)propanal, le 3-(4-(tert-butyl)-6-méthylcyclohex-3-én-1-yl)propanai, le 3-(4-isopropyl-6-méthylcyclohex-3-én-1-yl)propanal, le 3-(4-(sec-butyl)-6-méthylcyclohex-3-én-1-yl)propanal, le 3-(4-isobutyl-6-méthylcyclohex-3-én-1-yl)propanal, le 3-(4-(tert-butyl)-2-méthylcyclohex-3-én-1-yl)propanal, le 3-(4-isopropyl-2-méthylcyclohex-3-én-1-yl)propanal, le 3-(4-(sec-butyl)-2-méthylcyclohex-3-én-1-yl)propanal et le 3-(4-isobutyl-2-méthylcyclohex-3-én-1-yl)propanal.

7. Composition de parfum selon l'une des revendications 1 à 7 qui est exempte d'alcanals substitués par aryle, en particulier de propanals substitués par aryle, qui sont non substitués sur le cycle aryle au niveau d'une position ortho par rapport au substituant portant la fonctionnalité aldéhyde, en particulier exempte de 3-(4-tert-butylphényl)-2-méthylpropanal.

8. Produit de consommation, en particulier produit de soin personnel ou produit d'entretien ménager, comprenant une composition de parfum selon l'une des revendications 1 à 7.
